# EUROPÄISCHE PATENTSCHRIFT

(11) **EP 1 708 634 B1**
(45) Veröffentlichungstag und Bekanntmachung des Hinweises auf die Patenterteilung: **12.03.2008**
(21) Anmeldenummer: 04821236.9
(22) Anmeldetag: 04.11.2004
(51) Int. Cl.: A61B 18/14

(54) **CHIRURGISCHE PINZETTE**
SURGICAL FORCEPS
PINCE CHIRURGICALE

(30) Priorität: 28.01.2004 DE 102004004149
(43) Veröffentlichungstag der Anmeldung: 11.10.2006
(73) Patentinhaber: AESCULAP AG & Co. KG, 78532 Tuttlingen (DE)
(72) Erfinder: SCHWEITZER, Tom, 78532 Tuttlingen (DE); NESPER, Markus, 78532 Tuttlingen (DE); WEISSHAUPT, Dieter, 78194 Immendingen (DE); KUPFERSCHMID, Bernhard, 78576 Emmingen-Liptingen (DE); FISCHER, Manfred, 73479 Ellwangen (DE)
(74) Vertreter: Hoeger, Stellrecht & Partner Patentanwälte
(86) Internationale Anmeldenummer: PCT/EP2004/012457
(87) Internationale Veröffentlichungsnummer: WO 2005/072633

(56) Entgegenhaltungen:
- EP-A1- 0 546 209
- WO-A-95/07662
- DE-A1- 2 734 847
- DE-A1- 19 800 732
- DE-A1- 19 852 682
- DE-A1- 19 910 457
- US-A- 5 913 874
- US-A- 5 957 937

## Beschreibung

Die Erfindung betrifft eine chirurgische Pinzette mit einem länglichen Schaft, in dem zwei Pinzettenschenkel längsverschieblich gelagert sind, deren freie, aus dem Schaft austretende Enden durch die Längsverschiebung gegenüber dem Schaft in ihrem gegenseitigen Abstand veränderbar sind, und mit einem Griffteil, an dem der Schaft gehalten ist und welches ein Betätigungselement zur Längsverschiebung der Schenkel relativ zum Schaft aufweist, wobei der Schaft mit den darin gelagerten Schenkeln eine lösbar mit dem Griffteil verbundene Baueinheit bildet und wobei die Schenkel elektrische Leiter bilden oder im Inneren des Schaftes mit elektrischen Leitern verbunden sind, die am griffteilseitigen Ende des Schaftes isoliert aus dem Schaft austreten.

Eine solche Pinzette ist beispielsweise in der DE 27 34 847 A1 beschrieben.

Das Dokument DE 19 739 032 C2 offenbart ebenfalls eine gattungsgemäße Pinzette.

Ausgehend von diesem Stand der Technik liegt dem Anmeldegegenstand die Aufgabe zugrunde, eine chirurgische Pinzette zur Verfügung zu stellen, die besonders einfach aufgebaut ist und zumindest teilweise die Verwendung im Einmal- oder Wegwerfbetrieb ermöglicht.

Diese Aufgabe wird bei einer chirurgischen Pinzette der eingangs beschriebenen Art dadurch gelöst, dass die elektrischen Leiter von der griffteilseitigen Austrittsstelle des Schaftes in einem sich unmittelbar an den Schaft anschließenden Anschlusskabel verlaufen, das ebenfalls Teil der Baueinheit ist, die als Wegwerfteil ausgebildet ist. Diese Baueinheit kann bei Bedarf mit einem Griffteil verbunden und wieder von diesem gelöst werden, so daß die Baueinheit als Wegwerf- oder Einmalteil ausgebildet werden kann, während das Griffteil wiederholt eingesetzt werden kann.

Vorteilhaft ist es, wenn der Schaft in einer Lagerhülse längsverschieblich gelagert ist, die lösbar mit dem Griffteil verbindbar ist und an der die Schenkel unverschieblich festgelegt sind, und wenn das Betätigungselement zur Verschiebung des Schaftes mit diesen in Wirkverbindung steht, wenn die Lagerhülse am Griffteil festgelegt ist. Das Griffteil wird über die Lagerhülse also unverschiebbar mit den Schenkeln verbunden, die an der Lagerhülse festgelegt sind, und gegenüber dieser Einheit aus Griffteil, Lagerhülse und Schenkeln wird der Schaft in Längsrichtung verschoben, um dadurch die Schließ- und / oder Spreizbewegung der Schenkel zu erzeugen.

Die Schenkel können an der Lagerhülse vorzugsweise durch ein Klemmelement festgelegt sein.

Bei einer bevorzugten Ausführungsform ist vorgesehen, daß das Betätigungselement einen Mitnahmevorsprung aufweist, der bei am Griffteil festgelegter Lagerhülse in eine Mitnahmevertiefung des Schaftes eingreift. Beim Aufsetzen der Baueinheit auf das Griffteil tritt das Betätigungselement bei dieser Ausgestaltung in die Mitnahmevertiefung des Schaftes ein, es ergibt sich dadurch eine formschlüssige Verbindung, die bei der Abnahme des Bauteils vom Griffteil einfach dadurch wieder gelöst werden kann, daß bei der Abnahme des Bauteils der Mitnahmevorsprung wieder aus der Mitnahmevertiefung austritt.

Besonders vorteilhaft ist eine Ausgestaltung, bei der vorgesehen ist, daß die Baueinheit mit dem Griffteil eine Schwenkverbindung bildet, um deren Schwenkachse die Baueinheit in eine Verbindungsstellung verschwenkbar ist, und daß die Baueinheit mit dem Griffteil in der Verbindungsstellung verriegelbar ist. Die Schwenkverbindung kann dabei vorzugsweise lösbar sein.

Insbesondere kann die Schwenkverbindung eine konzentrisch zur Schwenkachse verlaufende Lagerfläche und mindestens eine diese teilweise umgebenden Lagerhaken umfassen. Es ist für den Benutzer dann sehr einfach, die Baueinheit mittels des Lagerhakens unter Ausbildung der Schwenkverbindung in die Lagerfläche einzuhaken und durch Verschwenkung um die dadurch gebildete Schwenkachse in die Verbindungsstellung zu bringen, in der Griffteil und Baueinheit miteinander verriegelt werden.

Die Verriegelung läßt sich besonders vorteilhaft ausgestalten, wenn zur Verriegelung der Baueinheit mit dem Griffteil ein federbelasteter Rasthebel vorgesehen ist, an dem die Baueinheit beim Bewegen in die Verbindungsstellung federnd vorbeigleitet. Der Zusammenbau erfolgt also einfach durch Einhängen und Verschwenken der Baueinheit bis in die Verbindungsstellung, dabei erfolgt automatisch eine Verriegelung, die nur durch Betätigung des federbelasteten Rasthebels wieder aufgehoben werden kann.

Die Schenkel können als Streifen oder Stäbe aus einem elastischen Material ausgebildet sein, insbesondere können sie aus Metall bestehen. Beispielsweise können die Schenkel als Federdrähte ausgebildet sein, die sich durch die gesamte Baueinheit hindurch erstrecken.

Es ist vorteilhaft, wenn der Schaft aus Kunststoff besteht. Einerseits ist es dadurch möglich, eine sehr billige Baueinheit aufzubauen, andererseits stellt der Kunststoff sicher, daß die in ihm längsverschieblich gelagerten Schenkel elektrisch voneinander isoliert sind. Wenn die Pinzette also als mono- oder bipolare Pinzette zur Koagulation von Gewebe eingesetzt wird, können die Schenkel als Elektroden verwendet werden, ohne daß eine gesonderte Isolation der Schenkel im Inneren des Schaftes notwendig ist.

Bei einer besonders bevorzugten Ausführungsform ist vorgesehen, daß der Schaft aus zwei an Anlageflächen aneinander anliegenden Hälften besteht, die zueinander ausgerichtete, in Längsrichtung verlaufende Nuten in ihren einander zugewandten Anlageflächen aufweisen, die gemeinsam Längskanäle zur Aufnahme je eines Schenkels bilden. Die Schenkel sind also in diesen Längskanälen aufgenommen und geführt, dabei sind diese Längskanäle zu beiden Seiten der flächig aneinanderliegenden Anlageflächen angeordnet.

Günstig ist es dabei, wenn die beiden Hälften durch eine elastische Rast- oder Schnappverbindung miteinander verbunden sind. Zur Montage genügt es dann, in eine Hälfte die beiden Schenkel einzulegen und die andere Hälfte aufzulegen und durch einen leichten Druck mit der ersten Hälfte zu verbinden, weitere Montageschritte sind nicht notwendig.

Vorteilhaft ist es, wenn die Schenkel an der Austrittsstelle aus dem Schaft zu ihren freien Enden hin divergieren und mit ihren einander abgewandten Außenseiten federnd an Schließflächen des Schaftes anliegen. Beim Vorschieben des Schaftes in Längsrichtung in Richtung auf die freien Enden der Schenkel hin werden dadurch die beiden Schenkel federnd gegeneinander gedrückt, es erfolgt also eine Schließbewegung.

Es kann weiterhin vorgesehen sein, daß die Schenkel an der Austrittsstelle aus dem Schaft mit ihren einander zugewandten Innenseiten an Aufspreizflächen des Schaftes anliegen. Diese führen dazu, daß beim Zurückziehen des Schaftes längs der Schenkel diese aufgespreizt werden, es erfolgt also eine Öffnungsbewegung der Schenkel. Es ist dadurch möglich, mit dem beschriebenen Instrument sowohl die Schenkel bewußt zu schließen als auch bewußt zu öffnen, so daß das Instrument sowohl zum Greifen als auch zum Aufspreizen eingesetzt werden kann.

Die Schließflächen und / oder die Aufspreizflächen des Schaftes sind vorzugsweise in Längsrichtung des Schaftes zu den Schenkeln hin konvex gekrümmt, so daß diese Flächen nur über einen kurzen Längenbereich an den Schenkeln anliegen und sich damit auch an die unterschiedliche Neigung der Schenkel im Anlagebereich anpassen.

Weiterhin kann vorgesehen sein, daß den Schließflächen und / oder den Aufspreizflächen ein Abschnitt eines die Schenkel aufnehmenden Längskanales vorgelagert ist, der breiter ist als die darin verlaufenden Schenkel. Dadurch können in diesem verbreiterten Abschnitt die Schenkel quer zu ihrer Längsrichtung verlagert werden, so daß auch unterschiedlich geneigte Schenkelabschnitte in diesen Bereich aufgenommen werden können.

Es ist günstig, wenn die Schenkel im Bereich ihrer Austrittsstelle aus dem Schaft nach außen ausgebaucht sind. Dies führt dazu, daß die Divergenz der Schenkel im Austrittsbereich relativ groß ist und daß trotzdem die freien Enden der Schenkel nicht allzu weit voneinander entfernt sind. Dadurch wird das Übersetzungsverhältnis zwischen der Längsverschiebung des Schaftes einerseits und der Verschwenkbewegung der freien Enden des Schenkels andererseits beeinflußt, je stärker die Schenkel im Austrittsbereich divergieren, desto stärker werden die Schenkel bei einer bestimmten Verschiebung des Schaftes verschwenkt.

Insbesondere für die Verwendung der Pinzette zur Koagulation können die Schenkel elektrische Leiter bilden oder im Inneren des Schaftes mit elektrischen Leitern verbunden sein, wobei diese elektrischen Leiter vorzugsweise am griffteilseitigen Ende des Schaftes isoliert aus dem Schaft austreten. Es können also die Schenkel selbst, die beispielsweise als Federdrähte ausgebildet sind, oder aber mit den Schenkeln im Inneren des Schaftes verbundene elektrische Leiter ohne weitere äußere Verbindungsstelle am griffteilseitigen Ende des Schaftes isoliert aus dem Schaft austreten, in diesem Bereich ist kein besonderer abnehmbarer Anschluß notwendig. Dadurch werden die Sichtverhältnisse in diesem Bereich wesentlich verbessert, außerdem ergibt sich ein sehr einfacher Aufbau der Baueinheit.

Insbesondere können die elektrischen Leiter von der griffteilseitigen Austrittsstelle des Schaftes in einem sich unmittelbar an den Schaft anschließenden Anschlußkabel verlaufen. Der Schaft setzt sich also in einem Anschlußkabel fort, und dieses Anschlußkabel bildet dann auch einen Teil der Baueinheit.

Bei einer bevorzugten Ausführungsform ist vorgesehen, daß der Schaft zusätzlich zu den Schenkeln einen Flüssigkeitskanal aufnimmt, der im griffteilseitigen Ende des Schaftes in diesen eintritt. Dieser Flüssigkeitskanal kann bis zum vorderen Ende des Schaftes in diesem verlaufen und tritt dann im Bereich der freien Enden des Schenkels aus, so daß in diesem Bereich beispielsweise Spülflüssigkeit zugeführt werden kann.

Auch in diesem Falle ist es günstig, wenn der Flüssigkeitskanal von dem griffteilseitigen Ende des Schaftes an zusammen mit mit den Schenkeln verbundenen elektrischen Leitern in einem anschließenden Anschlußkabel verläuft, dabei kann dieses Kabel einen gemeinsamen Mantel aufweisen, der sowohl die elektrischen Leiter als auch den Flüssigkeitskanal aufnimmt, es wäre aber auch möglich, ein Kabel mit getrennten Mänteln für die elektrischen Leiter und den Flüssigkeitskanal vorzusehen.

Die vorstehend beschriebene Ausgestaltung des Bauteils macht dies besonders geeignet zur Verwendung als Einmal- oder Wegwerfteil, das heißt für jeden Einsatz wird ein und dasselbe Griffteil verwendet, das jeweils mit eine neuen Baueinheit bestückt wird. Dadurch ist es möglich, diese Baueinheit hinsichtlich der sterilen Aufbereitung und der Prüfung der elektrischen Eigenschaften in einfacher Weise vorzubereiten und dann die in dieser Weise vorgeprüfte Baueinheit erst im Operationssaal mit dem Griffteil zu verbinden und dort einzusetzen.

Die nachfolgende Beschreibung bevorzugter Ausführungsformen der Erfindung dient im Zusammenhang mit der Zeichnung der näheren Erläuterung. Es zeigen:

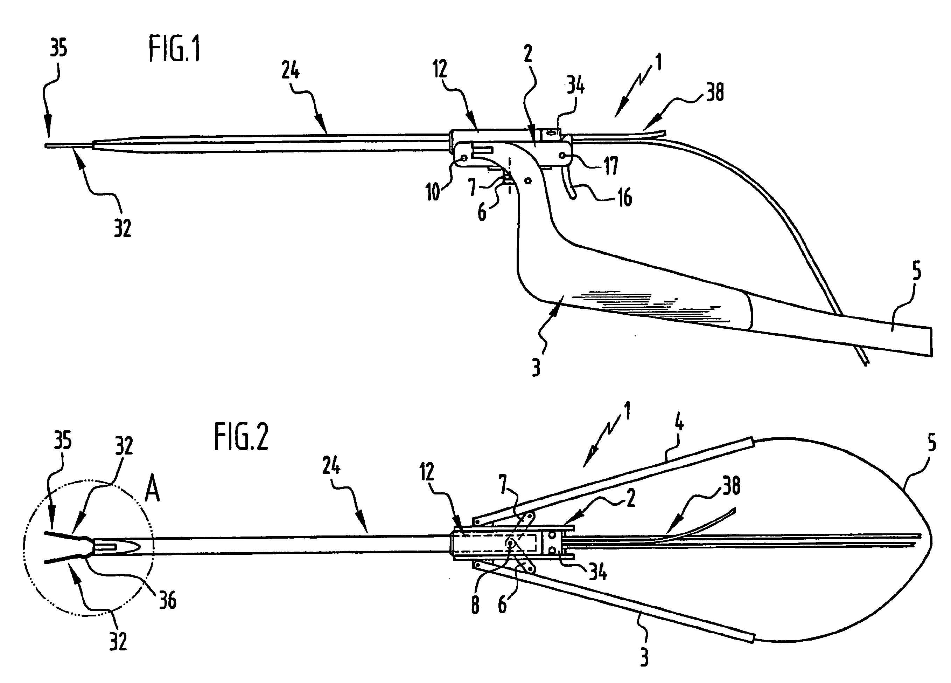

- Figur 1:: eine Seitenansicht einer chirurgischen Pinzette mit einem Griffteil und einer lösbar mit diesem verbundenen Baueinheit aus Schaft, Schenkeln und einem Anschlußkabel;
- Figur 2:: eine Draufsicht auf das chirurgische Instrument der Figur 1;

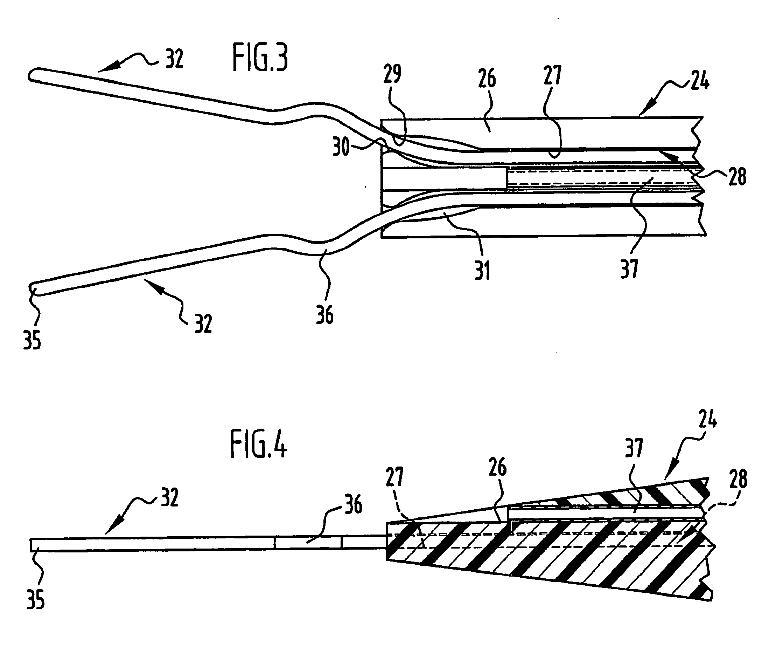

- Figur 3:: eine vergrößerte Teilansicht des Bereiches A in Figur 2 bei abgenommener oberer Hälfte des Schaftes;

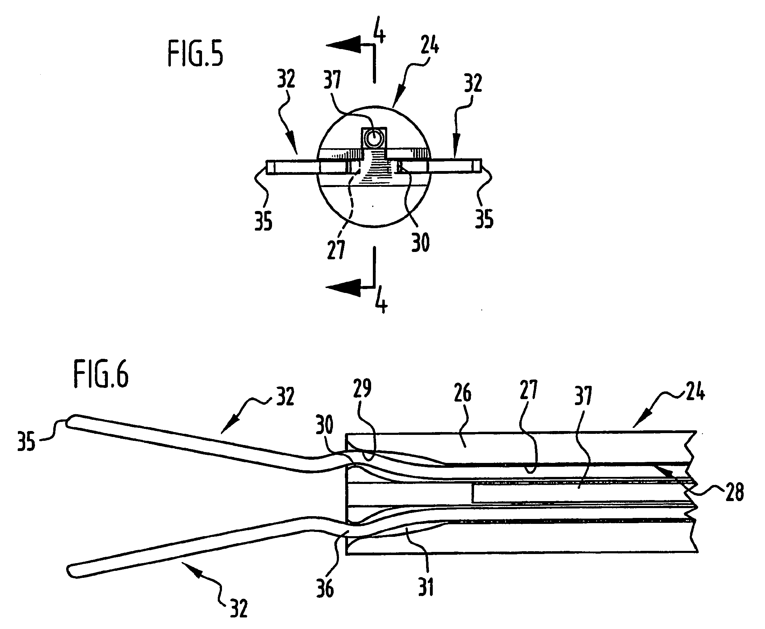

- Figur 4:: eine Längsschnittansicht des Bereiches A in Figur 2 längs Linie 4-4 in Figur 5;
- Figur 5:: eine Vorderansicht des Instrumentes der Figuren 1 und 2;
- Figur 6:: eine Ansicht ähnlich Figur 3 mit relativ zu den Schenkeln vorgeschobenem Schaft;

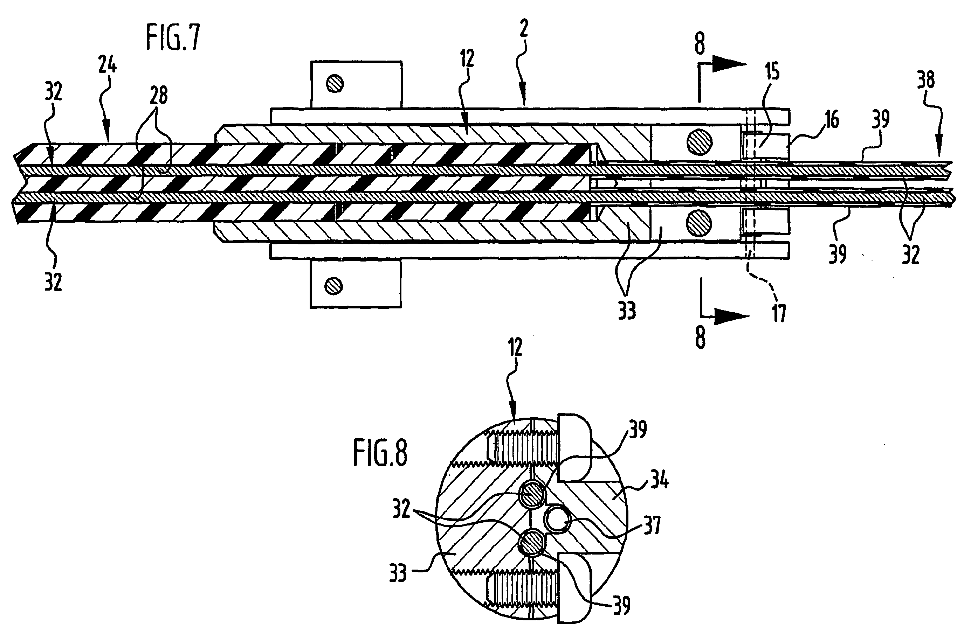

- Figur 7:: eine Längsschnittansicht der Baueinheit aus Schaft, Schenkeln und Anschlußkabel am griffteilseitigen Ende der Baueinheit;
- Figur 8:: eine Schnittansicht längs Linie 8-8 in Figur 7;
- Figur 9:: eine Seitenansicht der Baueinheit in Verbindungsstellung mit dem Griffteil und
- Figur 10:: eine Ansicht ähnlich Figur 9 bei vom Griffteil weggeschwenkter Baueinheit.

Das in der Zeichnung dargestellte chirurgische Instrument 1 ist als bipolare Koagulationspinzette ausgebildet. Es versteht sich aber, daß auch andere pinzettenartige Instrumente in ähnlicher Weise aufgebaut sein können. Dabei können diese Pinzetten sowohl zur Koagulation dienen als auch zum Greifen oder Aufspreizen, und die Arbeitsflächen der Pinzette am freien Ende der Pinzettenschenkel können dabei sehr unterschiedlich ausgestaltet sein, gegebenenfalls können diese auch ohne elektrische Zusatzfunktionen eingesetzt werden.

Das Instrument 1 weist eine Halterung 2 auf, an der auf gegenüberliegenden Seiten um eine senkrechte Achse verschwenkbar zwei Handgriffe 3, 4 gelagert sind, die an ihren freien Enden über eine im wesentlichen U-förmig verlaufende Bügelfeder 5 miteinander verbunden sind. Gegen die Wirkung der Bügelfeder 5 können die beiden Handgriffe um ihre Schwenklagerung an der Halterung 2 herum gegeneinander verschwenkt werden. Die beiden Handgriffe 3, 4 sind jeweils verschwenkbar mit zwei einarmigen Hebeln 6, 7 verbunden, die mit ihrem anderen Ende verschwenkbar miteinander verbunden sind, die beiden Hebel 6, 7 bilden also einen Kniehebel zwischen den beiden Handgriffen 3, 4 aus, der beim Zusammendrücken der Handgriffe 3, 4 zusammengefaltet wird und sich beim Auseinanderschwenken der Handgriffe 3, 4 streckt. Die Hebel 6, 7 tragen im Bereich ihrer gelenkigen Verbindung einen nach oben abstehenden Mitnehmerstift 8, der die Halterung 2 im Bereich eines Langloches 9 durchsetzt und nach oben über die Halterung 2 hervorsteht. Dieser Mitnehmerstift 8 kann durch die Verschwenkbewegung der Handgriffe 3, 4 in dem Langloch 9 geführt hin- und hergeschoben werden.

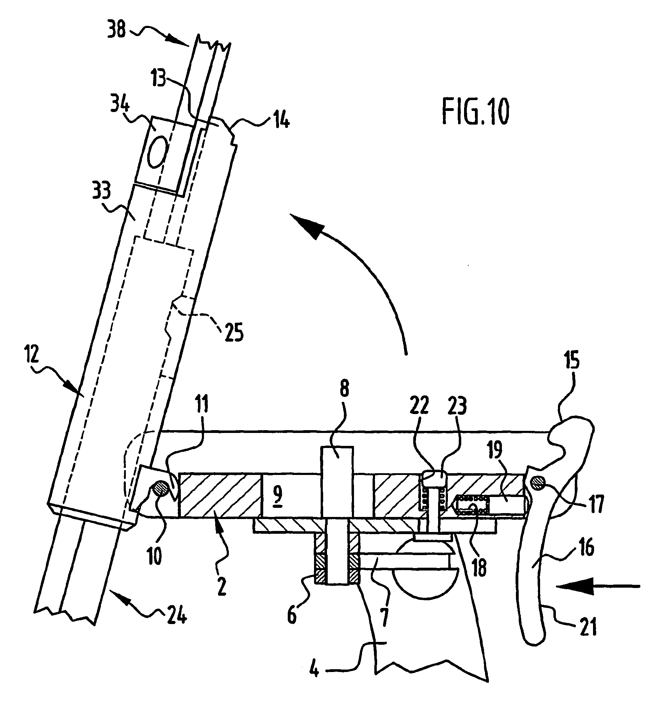

Am vorderen Ende der Halterung 2 ist eine quer zur Verschieberichtung des Mitnehmerstiftes 8 verlaufende Lagerwelle 10 angeordnet, die als Gegenlager für einen Lagerhaken 11 dient, der an einer Lagerhülse 12 nach unten abstehend angeordnet ist und zur Vorderseite der Lagerhülse 12 hin offen ist. Die Lagerhülse 12 kann im wesentlichen quer zur Ausdehnung der Halterung 2 von oben her so auf die Halterung aufgesetzt werden, daß der Lagerhaken 11 die Lagerwelle 10 von oben beidseitig umgreift und die Lagerhülse 12 somit auf der Lagerwelle 10 abstützt (Figur 10), dadurch wird eine Schwenklagerung für die Lagerhülse 12 an der Halterung 2 ausgebildet. Um die durch die Lagerwelle 10 gebildete Schwenkachse kann die Lagerhülse 12 entgegen der Richtung des Pfeiles A in Figur 10 in eine Verbindungsstellung verschwenkt werden, in der die Lagerhülse 12 auf die Halterung 2 aufgelegt ist und im wesentlichen parallel zu dieser verläuft.

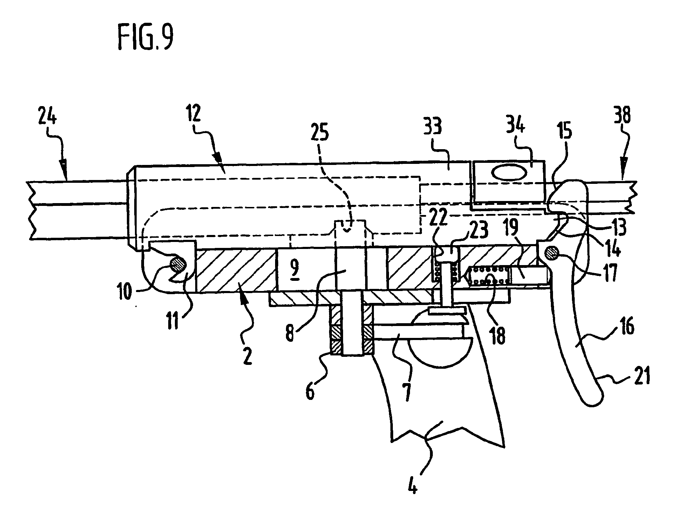

An dem dem Lagerhaken 11 gegenüberliegenden Ende der Lagerhülse 12 ist eine nach hinten vorspringende Rastnase 13 angeordnet, die beim Verschwenken der Lagerhülse 12 in die in Figur 9 dargestellte Verbindungsstellung mit einer schrägen Aufgleitfläche 14 an einer ebenfalls schrägen Aufgleitfläche 15 eines Rasthebels 16 entlanggleitet, welcher am hinteren Ende der Halterung 2 um eine parallel zur Lagerwelle 10 verlaufende Schwenkachse 17 schwenkbar gelagert ist. Durch einen federbelasteten, in der Halterung 2 in einer Bohrung 18 längsverschieblich gelagerten Stift 19, der sich an dem Rasthebel 16 abstützt, wird der Rasthebel 16 in eine Ruhestellung verschwenkt, in der der Rasthebel 16 gegen die Rastnase 13 der Lagerhülse 12 verschwenkt wird, also in die Bewegungsbahn der Rastnase 13 hinein, die diese beim Verschwenken der Lagerhülse 12 in die Verbindungsstellung durchläuft. Beim Verschwenken in die Verbindungsstellung wird durch die Anlage der beiden Aufgleitflächen 14 und 15 der Rasthebel 16 gegen die Wirkung des federbelasteten Stiftes 19 verschwenkt und schwenkt dann beim Erreichen der Verbindungsstellung wieder zurück, so daß die Rastnase 13 in einen Rastrücksprung 20 des Rasthebels 16 eingreift. Dadurch wird die Lagerhülse 12 an der Halterung 2 in der Verbindungsstellung verrastet (Figur 9). Eine Freigabe der Lagerhülse 12 von der Halterung 2 ist nur möglich, wenn der Rasthebel 16 durch einen Druck auf eine Grifffläche 21 entgegen der Wirkung des federbelasteten Stiftes 19 verschwenkt wird, wie dies durch den Pfeil B in Figur 10 angedeutet wird.

In der Halterung 2 ist in einer senkrechten Aufnahmebohrung 22 ein federbelasteter Auswerferstift 23 angeordnet, der bei entspannter Feder geringfügig nach oben über die Halterung 2 hervorsteht, der aber von der Lagerhülse 12 gegen die Federwirkung in die Aufnahmebohrung 22 hineingeschoben wird, wenn die Lagerhülse 12 mit der Halterung 2 in Verbindungsstellung verriegelt ist (Figur 9). Dadurch wird einerseits das Spiel zwischen Lagerhülse und Halterung beseitigt, andererseits wird bei einer Lösebetätigung des Rasthebels 16 die Lagerhülse 12 geringfügig aus der Verbindungsstellung in Richtung des Pfeiles A in Figur 10 verschwenkt, so daß beim Zurückfedern des Rasthebels 16 dieser die Rastnase 13 nicht mehr rastend umgreifen kann, sondern an dieser anliegt, die Verriegelung bleibt also geöffnet, so daß der Benutzer aus dieser entriegelten Stellung die Lagerhülse 12 in Richtung des Pfeiles A in eine aufrechte Stellung verschwenken und dann von der Halterung 2 abnehmen kann.

Die Lagerhülse 12 nimmt längsverschieblich einen Schaft 24 auf, der in der Lagerhülse 12 geführt ist und an der Vorderseite, also an der Seite des Lagerhakens 11, aus dieser hervorsteht. Die Lagerhülse 12 ist an ihrer Unterseite durchbrochen, und in diesem Bereich ragt der Mitnehmerstift 8 in den Innenraum der Lagerhülse 12 hinein, wenn die Lagerhülse 12 in der Verbindungsstellung mit der Halterung 2 verriegelt ist. Der Mitnehmerstift 8 taucht dabei in eine Mitnehmervertiefung 25 des Schaftes 24 ein, so daß bei einer Verschiebung des Mitnehmerstiftes 8 im Langloch 9 der Schaft 24 mitgenommen und in gleicher Weise relativ zu der Lagerhülse 12 verschoben wird.

Der Schaft 24 besteht aus zwei gleich aufgebauten Hälften, die längs ebener, einander zugewandter Anlageflächen 26 flächig aneinander anliegen und die mittels einer in der Zeichnung nicht dargestellten Rast- oder Schnappverbindung miteinander verbunden werden können. In beiden Hälften sind in die Anlageflächen 26 parallele Nuten 27 eingearbeitet, die Nuten der beiden Hälften sind so miteinander ausgerichtet, daß diese Nuten der beiden Hälften gemeinsam zwei im wesentlichen parallel durch den gesamten Schaft hindurch verlaufende Längskanäle 28 ausbilden. Beide Längskanäle 28 laufen am Vorderen Ende des Schaftes 24 schräg auseinander. Die beiden Seitenflächen der Längskanäle 28 bilden dabei einander gegenüberliegende Führungsflächen aus, nämlich an der Außenseite jeweils eine Schließfläche 29 und an der Innenseite jeweils eine Aufspreizfläche 30. Sowohl die Schließflächen 29 als auch die Aufspreizflächen 30 sind zum jeweiligen Längskanal 29 hin konvex ausgebaucht. Die Längskanäle 28 sind in dem sich unmittelbar an die Schließfläche 29 und die Aufspreizfläche 30 in Richtung auf die Halterung 2 anschließenden Abschnitt 31 verbreitert ausgebildet, das heißt der Abstand der Seitenwände ist in diesem Abschnitt 31 größer als im übrigen Bereich der Längskanäle 28.

In jeden der beiden Längskanäle 28 ist ein Schenkel 32 eingelegt, dieser Schenkel 32 kann als Federdraht ausgebildet sein oder als schmales Band aus einem elastischen Metall, beide Schenkel 32 sind dabei gleich aufgebaut und spiegelverkehrt in die Längskanäle 28 eingelegt. Die Breite der Schenkel 32 entspricht im wesentlichen der Breite der Längskanäle 28 und dem Abstand der Schließfläche 29 von der Aufspreizfläche 30, sie ist jedoch geringer als die Breite des verbreiterten Abschnittes 31 im Längskanal 28. Die Schenkel 32 treten durch den verschlossenen Boden 33 der Lagerhülse 12 hindurch und sind durch ein Klemmelement 34, welches mit der Halterung 2 verschraubt ist, fest mit der Halterung 2 verbunden, so daß sie relativ zur Halterung 2 in Längsrichtung unverschieblich sind. Dadurch verschiebt sich der Schaft 24 bei Betätigung der Handgriffe 3 relativ zu den beiden Schenkeln 32, und dies führt zu einer Verschwenkbewegung der aus dem Schaft 24 austretenden freien Enden 35 der Schenkel 32, diese werden nämlich gegeneinander verschwenkt. Beim Vorschieben des Schaftes 24 werden die beiden freien Enden gegeneinander verschwenkt, also geschlossen, beim Zurückziehen des Schaftes 24 dagegen auseinandergeschwenkt oder aufgespreizt. Diese Schwenkbewegung wird dadurch unterstützt, daß die freien Enden 35 im Austrittsbereich aus dem Schaft 24 beide nach außen ausgebauchte Abschnitte 36 aufweisen, so daß die Schenkel 32 im Austrittsbereich zunächst unter einem relativ großen Winkel gegenüber der Längsachse des Schaftes aus diesem austreten.

Im Schaft 24 ist parallel zu den Längskanälen 28 verlaufend ein diesen durchsetzender Flüssigkeitskanal 37 angeordnet, dieser kann beispielsweise gebildet werden durch ein in den Schaft 24 eingelegtes Röhrchen.

An der Rückseite der Lagerhülse 12 treten sowohl die beiden Schenkel 32 als auch der Flüssigkeitskanal 37 unmittelbar in ein Anschlußkabel 38 ein, welches mit der Lagerhülse eine Baueinheit bildet und sich in Längsrichtung an diese anschließt. Die Schenkel 32 sind dabei bis in den Eintritt in die Längskanäle 28 des Schaftes 24 von einem Isoliermantel 39 umgeben, so daß die beiden Schenkel 32 über ihre gesamte Länge elektrisch voneinander isoliert sind.

Das Anschlußkabel führt zu einer Versorgungseinheit, durch die die beiden Schenkel 32 mit einer Spannungsquelle einer Hochfrequenzeinheit verbunden werden, der Flüssigkeitskanal 37 mit einer Flüssigkeitsquelle, die beispielsweise Spülflüssigkeit liefert. Die Baueinheit aus Schaft, Schenkeln, Lagerhülse und Anschlußkabel ist so einfach aufgebaut, daß diese Baueinheit als Einwegteil verwendet werden kann, alle Prüfungen hinsichtlich der elektrischen Sicherheit und der Sterilität können fabrikseitig vorgenommen werden, die Baueinheit wird in vorbereitetem Zustand in Sterilverpackung an den Einsatzort geliefert und kann dort in der beschriebenen Weise mit der Halterung 2 verbunden werden, die wiederholt einsetzbar ist. Die beiden Schenkel 32 können als Elektroden eines bipolaren Instrumentes eingesetzt werden, das Instrument kann dann beispielsweise zur Koagulation dienen.

## Patentansprüche

1. Chirurgische Pinzette mit einem länglichen Schaft (24), in dem zwei Pinzettenschenkel (32) längsverschieblich gelagert sind, deren freie, aus dem Schaft (24) austretende Enden durch die Längsverschiebung gegenüber dem Schaft (24) in ihrem gegenseitigen Abstand veränderbar sind, und mit einem Griffteil (2), an dem der Schaft (24) gehalten ist und welches ein Betätigungselement (8) zur Längsverschiebung der Schenkel (32) relativ zum Schaft aufweist, wobei der Schaft (24) mit den darin gelagerten Schenkeln (32) eine lösbar mit dem Griffteil (2) verbundene Baueinheit bildet und wobei die Schenkel (32) elektrische Leiter bilden oder im Inneren des Schaftes (24) mit elektrischen Leitern verbunden sind, die am griffteilseitigen Ende des Schaftes (24) isoliert aus dem Schaft (24) austreten, **dadurch gekennzeichnet, dass** die elektrischen Leiter von der griffteilseitigen Austrittsstelle des Schaftes (24) in einem sich unmittelbar an den Schaft (24) anschließenden Anschlusskabel (38) verlaufen, das ebenfalls Teil der Baueinheit ist, die als Wegwerfteil ausgebildet ist.

2. Pinzette nach Anspruch 1, **dadurch gekennzeichnet, daß** der Schaft (24) in einer Lagerhülse (12) längsverschieblich gelagert ist, die lösbar mit dem Griffteil (2) verbindbar ist und an der die Schenkel (32) unverschieblich festgelegt sind, und dass das Betätigungselement (8) zur Verschiebung des Schaftes (24) mit diesem in Wirkverbindung steht, wenn die Lagerhülse (12) am Griffteil (2) festgelegt ist.

3. Pinzette nach Anspruch 2, **dadurch gekennzeichnet, dass** die Schenkel (32) an der Lagerhülse (12) durch ein Klemmelement (34) festgelegt sind.

4. Pinzette nach Anspruch 2 oder 3, **dadurch gekennzeichnet, dass** das Betätigungselement (8) einen Mitnahmevorsprung aufweist, der bei am Griffteil (2) festgelegter Lagerhülse (12) in eine Mitnahmevertiefung (25) des Schaftes (24) eingreift.

5. Pinzette nach einem der voranstehenden Ansprüche, **dadurch gekennzeichnet, dass** die Baueinheit mit dem Griffteil (2) eine Schwenkverbindung bildet, um deren Schwenkachse (10) die Baueinheit in eine Verbindungsstellung verschwenkbar ist, und dass die Baueinheit mit dem Griffteil (2) in der Verbindungsstellung verriegelbar ist.

6. Pinzette nach Anspruch 5, **dadurch gekennzeichnet, dass** die Schwenkverbindung lösbar ist.

7. Pinzette nach Anspruch 6, **dadurch gekennzeichnet, dass** die Schwenkverbindung eine konzentrisch zur Schwenkachse (10) verlaufende Lagerfläche und mindestens einen diese teilweise umgebenden Lagerhaken (11) umfasst.

8. Pinzette nach einem der Ansprüche 5 bis 7, **dadurch gekennzeichnet, dass** zur Verriegelung der Baueinheit mit dem Griffteil (2) ein federbelasteter Rasthebel (16) vorgesehen ist, an dem die Baueinheit beim Bewegen in die Verbindungsstellung federnd vorbeigleitet.

9. Pinzette nach einem der voranstehenden Ansprüche, **dadurch gekennzeichnet, dass** die Schenkel (32) als Streifen oder Stäbe aus einem elastischen Material ausgebildet sind.

10. Pinzette nach Anspruch 9, **dadurch gekennzeichnet, dass** die Schenkel (32) aus Metall bestehen.

11. Pinzette nach einem der voranstehenden Ansprüche, **dadurch gekennzeichnet, dass** der Schaft (24) aus Kunststoff besteht.

12. Pinzette nach einem der voranstehenden Ansprüche, **dadurch gekennzeichnet, dass** der Schaft (24) aus zwei an Anlageflächen (26) aneinander anliegenden Hälften besteht, die zueinander ausgerichtete, in Längsrichtung verlaufende Nuten (27) in ihren einander zugewandten Anlageflächen (26) aufweisen, die gemeinsam Längskanäle (28) zur Aufnahme je eines Schenkels (32) bilden.

13. Pinzette nach Anspruch 12, **dadurch gekennzeichnet, dass** die beiden Hälften durch eine elastische Rast- oder Schnappverbindung miteinander verbunden sind.

14. Pinzette nach einem der voranstehenden Ansprüche, **dadurch gekennzeichnet, dass** die Schenkel (32) an der Austrittsstelle aus dem Schaft (24) zu ihren freien Enden (35) hin divergieren und mit ihren einander abgewandten Außenseiten federnd an Schließflächen (29) des Schaftes (24) anliegen.

15. Pinzette nach Anspruch 14, **dadurch gekennzeichnet, dass** die Schenkel (32) an der Austrittsstelle aus dem Schaft (24) mit ihren einander zugewandten Innenseiten an Aufspreizflächen (30) des Schaftes (24) anliegen.

16. Pinzette nach einem der Ansprüche 14 oder 15, **dadurch gekennzeichnet, dass** die Schließflächen (29) und / oder die Aufspreizflächen (30) in Längsrichtung des Schaftes (24) zu den Schenkeln (32) hin konvex gekrümmt sind.

17. Pinzette nach einem der Ansprüche 14 bis 16, **dadurch gekennzeichnet, dass** den Schließflächen (29) und / oder den Aufspreizflächen (30) ein Abschnitt (31) eines die Schenkel (32) aufnehmenden Längskanals (28) vorgelagert ist, der breiter ist als die darin verlaufenden Schenkel (32).

18. Pinzette nach einem der Ansprüche 14 bis 17, **dadurch gekennzeichnet, dass** die Schenkel (32) im Bereich ihrer Austrittsstelle aus dem Schaft (24) nach außen ausgebaucht sind.

19. Pinzette nach einem der voranstehenden Ansprüche, **dadurch gekennzeichnet, dass** der Schaft (24) zusätzlich zu den Schenkeln (32) einen Flüssigkeitskanal (37) aufnimmt, der am griffteilseitigen Ende des Schaftes (24) in diesen eintritt.

20. Pinzette nach Anspruch 19, **dadurch gekennzeichnet, dass** der Flüssigkeitskanal (37) von dem griffteilseitigen Ende des Schaftes (24) an zusammen mit den Schenkeln (32) oder mit diesen verbundenen elektrischen Leitern in einem anschließenden Anschlusskabel (38) verläuft.

## Claims

1. Surgical forceps with an elongate shaft (24) in which two forceps arms (32) are longitudinally displaceably mounted, the free ends of which issue from the shaft (24) and are variable in their mutual spacing by longitudinal displacement relative to the shaft (24), and with a gripping part (2) on which the shaft (24) is held and which has an actuating member (8) for the longitudinal displacement of the arms (32) relative to the shaft, wherein the shaft (24) and the arms (32) mounted therein together form a structural unit which is detachably connected to the gripping part (2) and wherein the arms (32) form electrical conductors or are connected inside the shaft (24) to electrical conductors which issue from the shaft (24) in an insulated manner at the gripping-part end of the shaft (24), **characterised in that** the electrical conductors extend from the exit point of the shaft (24) at the gripping-part end thereof in a connecting cable (38) which directly adjoins the shaft (24) and is also part of the structural unit, which is formed as a disposable part.

2. Forceps according to claim 1, **characterised in that** the shaft (24) is longitudinally displaceably mounted in a bearing sleeve (12) which is detachably connectable to the gripping part (2) and to which the arms (32) are non-displaceably fastened, and **in that** the actuating member (8) is operatively connected to the shaft (24) for the displacement thereof when the bearing sleeve (12) is fastened to the gripping part (2).

3. Forceps according to claim 2, **characterised in that** the arms (32) are fastened to the bearing sleeve (12) by a clamping member (34).

4. Forceps according to claim 2 or 3, **characterised in that** the actuating member (8) has a carrier projection which engages in a carrier recess (25) in the shaft (24) when the bearing sleeve (12) is fastened to the gripping part (2).

5. Forceps according to any one of the preceding claims, **characterised in that** the structural unit and the gripping part (2) form a pivoted connection, about the pivoting axis (10) of which the structural unit is pivotable into a connecting position, and **in that** the structural unit is lockable to the gripping part (2) in the connecting position.

6. Forceps according to claim 5, **characterised in that** the pivoted connection is disengageable.

7. Forceps according to claim 6, **characterised in that** the pivoted connection comprises a bearing surface extending concentrically with the pivoting axis (10) and at least one bearing hook (11) partially surrounding the bearing surface.

8. Forceps according to any one of claims 5 to 7, **characterised in that**, in order to lock the structural unit to the gripping part (2), a spring-loaded locking lever (16) is provided, past which the structural unit resiliently slides during its movement into the connecting position.

9. Forceps according to any one of the preceding claims, **characterised in that** the arms (32) are formed as strips or rods of a resilient material.

10. Forceps according to claim 9, **characterised in that** the arms (32) are made of metal.

11. Forceps according to any one of the preceding claims, **characterised in that** the shaft (24) is made of plastics.

12. Forceps according to any one of the preceding claims, **characterised in that** the shaft (24) consists of two halves which rest against one another on contact surfaces (26) and which have facing, longitudinally extending grooves (27) in their facing contact surfaces (26), said grooves (27) together forming longitudinal channels (28) for receiving a respective arm (32).

13. Forceps according to claim 12, **characterised in that** the two halves are connected to one another by a resilient locking or snap connection.

14. Forceps according to any one of the preceding claims, **characterised in that** the arms (32) diverge in the direction of their free ends (35) at the exit point from the shaft (24), and their remote outer surfaces rest resiliently against closing surfaces (29) of the shaft (24).

15. Forceps according to claim 14, **characterised in that** the facing inner surfaces of the arms (32) rest against opening surfaces (30) of the shaft (24) at the exit point from the shaft (24).

16. Forceps according to either one of claims 14 and 15, **characterised in that** the closing surfaces (29) and/or the opening surfaces (30) are convexly curved towards the arms (32) in the longitudinal direction of the shaft (24).

17. Forceps according to any one of claims 14 to 16, **characterised in that** a portion (31) of a longitudinal channel (28) receiving the arms (32) is arranged upstream of the closing surfaces (29) and/or the contact surfaces¹ (30) and is wider than the arms (32) extending in the longitudinal channel (28).
¹The German text states *Anlageflächen* (contact surfaces), but should presumably read *Aufspreizflächen* (opening surfaces).

18. Forceps according to any one of claims 14 to 17, **characterised in that** the arms (32) protrude outwards in the region of their exit point from the shaft (24).

19. Forceps according to any one of the preceding claims, **characterised in that**, in addition to the arms (32), the shaft (24) contains a liquid duct (37) which enters the shaft (24) at the gripping-part end thereof.

20. Forceps according to claim 19, **characterised in that** the liquid duct (37) extends from the gripping-part end of the shaft (24) into an adjoining connecting cable (38) together with the arms (32) or electrical conductors connected thereto.

## Revendications

1. Pincette chirurgicale comprenant un corps (24) allongé dans lequel sont montées en coulissement longitudinal, deux branches de pincette (32) dont la distance d'espacement réciproque des extrémités libres sortant du corps (24) varie sous l'effet du coulissement longitudinal par rapport au corps (24), la pincette comprenant également une partie de poignée (2) sur laquelle est maintenu le corps (24) et qui présente un élément d'actionnement (8) pour le coulissement longitudinal des branches (32) par rapport au corps, le corps (24) formant avec les branches (32) qui y sont montées, un module lié de manière démontable à la partie de poignée (2), et les branches (32) formant des conducteurs électriques ou étant reliées, à l'intérieur du corps (24), à des conducteurs électriques qui, à l'extrémité du corps (24) située du côté de la partie de poignée, sortent de manière isolée du corps (24),
**caractérisée en ce que** les conducteurs électriques, à partir de la zone de sortie du corps (24), du côté de la partie de poignée, s'étendent dans un câble de raccordement (38) qui se raccorde directement au corps (24) et fait également partie dudit module qui est réalisé en tant que pièce à jeter ou pièce à usage unique.

2. Pincette selon la revendication 1, **caractérisée en ce que** le corps (24) est monté en coulissement longitudinal dans une douille de palier (12) qui peut être liée de manière démontable à la partie de poignée (2) et sur laquelle sont fixées, de façon non coulissante, les branches (32), et **en ce que** l'élément d'actionnement (8) est en interaction avec le corps (24) pour le faire coulisser, lorsque la douille de palier (12) est fixée à la partie de poignée (2).

3. Pincette selon la revendication 2, **caractérisée en ce que** les branches (32) sont fixées à la douille de palier (12) par un élément de serrage (34).

4. Pincette selon la revendication 2 ou 3, **caractérisée en ce que** l'élément d'actionnement (8) présente une protubérance d'entraînement qui, lorsque la douille de palier (12) est fixée à la partie de poignée (2), s'engage dans un creux d'entraînement (25) du corps (24).

5. Pincette selon l'une des revendications précédentes, **caractérisée en ce que** ledit module forme avec la partie de poignée (2) une liaison de pivotement autour de l'axe de pivotement (10), de laquelle il est possible de faire pivoter le module dans une position de liaison, et **en ce que** le module peut être verrouillé avec la partie de poignée (2) dans la position de liaison.

6. Pincette selon la revendication 5, **caractérisée en ce que** la liaison de pivotement peut être interrompue.

7. Pincette selon la revendication 6, **caractérisée en ce que** la liaison de pivotement comprend une surface de palier s'étendant de manière concentrique à l'axe de pivotement (10), et au moins un crochet de palier (11) entourant celle-ci partiellement.

8. Pincette selon l'une des revendications 5 à 7, **caractérisée en ce que** pour verrouiller le module avec la partie de poignée (2), il est prévu un levier d'encliquetage (16) sollicité par ressort et sur lequel glisse le module lors du mouvement vers la position de liaison.

9. Pincette selon l'une des revendications précédentes, **caractérisée en ce que** les branches (32) sont réalisées sous forme de bandes ou de barres en un matériau élastique.

10. Pincette selon la revendication 9, **caractérisée en ce que** les branches (32) sont réalisées en métal.

11. Pincette selon l'une des revendications précédentes, **caractérisée en ce que** le corps (24) est réalisé en matière plastique.

12. Pincette selon l'une des revendications précédentes, **caractérisée en ce que** le corps (24) est constitué de deux moitiés qui sont en appui réciproque l'une sur l'autre au niveau de surfaces d'appui (26), et présentent, dans leurs surfaces d'appui (26) dirigées l'une vers l'autre, des rainures (27) mutuellement concordantes, qui s'étendent dans la direction longitudinale et forment en commun des canaux longitudinaux (28) destinés à recevoir chacun une branche (32).

13. Pincette selon la revendication 12, **caractérisée en ce que** les deux moitiés sont reliées l'une à l'autre par une liaison élastique à encliquetage ou enclenchement.

14. Pincette selon l'une des revendications précédentes, **caractérisée en ce que** les branches (32), au niveau de leur zone de sortie du corps (24), divergent vers leurs extrémités libres (35), et s'appuient de manière élastique, avec leurs côtés extérieurs opposés, contre des surfaces de fermeture (29) du corps (24).

15. Pincette selon la revendication 14, **caractérisée en ce que** les branches (32), au niveau de leur zone de sortie du corps (24), s'appuient, avec leurs côtés intérieurs dirigés l'un vers l'autre, sur des surfaces d'écartement (30) du corps (24).

16. Pincette selon l'une des revendications 14 ou 15, **caractérisée en ce que** les surfaces de fermeture (29) et/ou les surfaces d'écartement (30) sont courbées de manière convexe dans la direction longitudinale du corps (24), en direction des branches (32).

17. Pincette selon l'une des revendications 14 à 16, **caractérisée en ce que** les surfaces de fermeture (29) et/ou les surfaces d'écartement (30) sont précédées d'un tronçon (31) d'un canal longitudinal (28) recevant les branches (32), qui est plus large que les branches (32) qui s'y étendent.

18. Pincette selon l'une des revendications 14 à 17, **caractérisée en ce que** les branches (32) sont bombées vers l'extérieur dans la région de leur zone de sortie du corps (24).

19. Pincette selon l'une des revendications précédentes, **caractérisée en ce que** le corps (24) reçoit en plus des branches (32), un canal de liquide (37) qui s'engage dans le corps (24) au niveau de l'extrémité de celui-ci située du côté de la partie de poignée.

20. Pincette selon la revendication 19, **caractérisée en ce que** le canal de liquide (37), à partir de l'extrémité côté partie de poignée du corps (24), s'étend, en commun avec les branches (32) ou avec des conducteurs électriques reliés à celles-ci, dans un câble de raccordement (38) qui s'y raccorde.
